# EUROPEAN PATENT SPECIFICATION

(11) **EP 3 103 420 B1**
(45) Date of publication and mention of the grant of the patent: **08.11.2017**
(21) Application number: 15707404.8
(22) Date of filing: 30.01.2015
(51) Int. Cl.: A61F 5/56, A61C 7/36

(54) **MANDIBULAR ADVANCEMENT DEVICE**
MANDIBULÄRE VORSCHUBVORRICHTUNG
DISPOSITIF D'AVANCEMENT MANDIBULAIRE

(30) Priority: 06.02.2014 ES 201400094
(43) Date of publication of application: 14.12.2016
(73) Proprietor: Biotechnology Institute, I MAS D, S.L., 01005 Vitoria (ES)
(72) Inventor: ANITUA ALDECOA, Eduardo, E-01005 Vitoria (ES)
(74) Representative: Cueto, Sénida
(86) International application number: PCT/ES2015/070065
(87) International publication number: WO 2015/118201

(56) References cited:
- EP-A1- 1 658 823
- WO-A2-2013/049751
- CN-Y- 201 422 945
- DE-A1-102008 007 281
- DE-U1-202008 016 373
- US-B1- 6 325 064

## Description

### Field of the Invention

The invention relates to a mandibular advancement device intended to be placed in a person's mouth and cause a slight advancement of the lower jaw so as to avoid the obstruction of airways. The main application of this type of device is the treatment of sleep apnea. The disclosure also relates to a preparation method and kit for this device.

### Prior Art

While people are asleep, the muscles relax causing the airways to close and vibrate as air is passed. This vibration causes the noise known as snoring. Snoring, however, is not the only problem caused by the obstruction of the airways. This obstruction also causes insufficient ventilation of the lungs, leading to disturbed sleep and, potentially, to the person's breathing to stop for a moment (this being known as sleep apnea). More specifically, the apnea phenomenon takes place as follows: after snoring regularly for a period of time, the snoring is suddenly interrupted by a brief period of silence in which breathing momentarily stops; this period without breathing (apnea) ends with a snort or gasp, after which normal breathing is gradually resumed, and the snoring begins again. This pattern may be repeated frequently during the night. Sleep apnea may lead to health problems and even death, and is therefore a serious health problem. This problem often arises in those suffering from obesity or in middle-aged men, although other factors such as alcohol or tobacco consumption and the anatomy of the nasal passage are also influential.

Diverse treatments for apnea are known which seek to normalize the patient's breathing during sleep. On one hand, apnea can be treated by reducing the risk factors in the patient's life. On the other hand, diverse devices are available on the market, which are intended to act on the patient in order to reduce sleep apnea. One type of device is based on injecting compressed air through a mask worn by the patient whilst sleeping, to ensure the controlled and regular opening of the airways. Another type of device, known as a mandibular advancement device, is placed inside the patient's mouth and causes a slight forward movement of the mandible with respect to the upper maxillary bone, opening a bigger space at the back of the oral cavity, hence facilitating the passage of air from and to the pharynx. In extreme cases, apnea can also be treated by surgery if the aforementioned treatments are not effective.

Mandibular advancement devices are usually made up of a mouthpiece, which is custom-made for the patient, with an upper portion which is to be placed in the upper dental arch and a lower portion to be placed in the lower dental arch. Mandibular advancement mechanisms are connected between the upper portion and the lower portion, and are positioned and tightened so that the lower portion is kept further forward than the upper portion, compared with a rest position in which both portions are located above each other in accordance with the patient's normal bite.

Diverse types of mandibular advancement mechanisms are known in prior art. For example, there are metal mechanisms consisting of a lower part and an upper part connected to the lower portion and the upper portion of the mouthpiece respectively. The lower part and the upper part are interconnected in a longitudinally adjustable manner, for example via an adjustable threaded connection, which enables the position of the lower portion of the mouthpiece to be adjusted with respect to the upper portion. These mandibular advancement devices based on metal mechanisms usually present the drawback of being extremely rigid and uncomfortable for the patient.

Alternatively, plastic mandibular advancement mechanisms are also available, which generally consist of one or more plastic straps that are connected between the lower portion and the upper portion of the mouthpiece. In some cases, the mechanism is made up of a single strap laid in the form of a connection rod in the front area of the upper and lower portions, connecting them so that the mandible is brought forward with respect to the upper maxillary bone. In other cases, the mechanism consists of two straps, one on each side of the mouthpiece, the ends of which are connected from a side of the lower portion to the same side of the upper portion. An example of this second type of mandibular advancement mechanism is described in Patent Application No. US20120073582A1. In other cases, a single strap is provided whose ends are connected to the opposing sides of the lower portion and whose central area is supported on the front of the upper portion of the mouthpiece. In general, the devices fitted with plastic mandibular advancement mechanisms are more comfortable for the patient as they provide certain lateral and/or longitudinal flexibility allowing the patient to move his or her jaw slightly during sleep, whilst maintaining it properly forward with respect to the upper jaw, achieving greater comfort and adaptation for the patient. Other known mandibular advancement devices are disclosed, for example, in WO2013/049751 A2, US6325064 B1, EP1658823 A1, CN201422945, or DE202008016373 U1, on which the preamble of appended claim 1 is based. The invention aims to provide an improved mandibular advancement device, offering the patient even greater comfort whilst ensuring the same or better mandibular advancement for the treatment of sleep apnea.

### Brief Description of the Invention

The object of the invention is a mandibular advancement device as defined in independent claim 1. Preferred embodiments are defined in the dependent claims.
In particular, the device comprises a mouthpiece made up of an upper portion and a lower portion, where both portions include a front area intended to be placed in the area of the incisor and canine teeth, and two lateral areas intended to be placed in the area of the premolar and molar teeth. The device in accordance with the invention presents the peculiarity that it comprises at least one plastic strap (cord), at each end of which a slot is provided. The slot is connected articulately to a respective protrusion which protrudes transversely from the mouthpiece. The slot is closed and its length is longer than that of the diameter of a cylindrical portion of the protrusion, so that the cylindrical portion can move along the slot. The mandibular advancement device is prepared or assembled starting with a patient's mouthpiece. Preparation is commenced by placing the mouthpiece in a position previously established by the doctor, in which the lower portion of the mouthpiece is placed sufficiently forward with respect to the upper portion so that, if the mouthpiece were placed inside the patient in this position, the mouthpiece would cause sufficient mandibular advancement so as to prevent apnea. This position that has been previously established by the doctor will be referred to as the "treatment position" throughout this document. Once the mouthpiece has been arranged in the treatment position, the strap or straps are articulately fixed to the mouthpiece by coupling the outer ends of the strap slots with the respective lateral protrusions of the mouthpiece. In other words, when the mouthpiece is in the treatment position, the protrusions are at the outer ends of the strap slots. Then, in this treatment position (in which the lower portion of the mouthpiece is slightly forward with respect to the upper portion to treat the apnea), the straps prevent the lower portion from moving backwards but do allow it to move forward.

In consequence, when the device in accordance with the invention is placed onto the patient, it initially forces the patient to adopt the treatment position, i.e., the position defined by the doctor as ideal for treating the apnea. From this position, the device in accordance with the invention does not allow the mandible to move backwards due to the traction exercised by the straps. On the other hand, the patient is able to slightly move the mandible forward, as far as the protrusions can move along the grooves, allowing certain freedom of movement and comfort for the patient. The patient will also be able to open and close his or her mouth, due to the fact that the straps are articulately connected to the mouthpiece (and therefore the straps work like articulated links). Furthermore, the position of the protrusions within the slots also allows the patient to slightly move his or her jaw in a transversal direction. Therefore, by preventing the backward movement of the jaw, the device in accordance with the invention allows the patient's apnea to be effectively treated; at the same time, the freedom of movement in the rest of the directions increases comfort, reduces any dental pain that may occur as a result of prolonged use of the device, and, in short, increases the patient's acceptance of the apnea treatment.

In different embodiments, the device may include with two straps, one on each side of the mouthpiece, or one strap that extends from one side of the mouthpiece to the other. In any case, the articulated connections between the portions of the mouthpiece and the straps are carried out as explained, i.e., at the outer ends of the strap slots when the device is in the treatment position that has been pre-established by the doctor.

### Brief Description of the Figures

The details of the invention can be seen in the accompanying figures, which do not intend to limit the scope of the invention:
- Figure 1 shows a perspective view of a first embodiment of the device in accordance with the invention.
- Figure 2 shows a front elevation view of the device of Figure 1.
- Figure 3 shows a partial left side elevation view of the device in Figure 1.
- Figure 4 shows a cross-sectional view of the assembly of Figure 3 as per section plane IV-IV.
- Figure 5 shows a side view of the device of Figure 1, in an initial position or treatment position.
- Figure 6 shows the device of Figure 5 in a second position in which the lower portion of the mouthpiece has slightly moved forward.
- Figure 7 shows the device of Figure 5 in a third position in which the lower portion of the mouthpiece has moved forward to a greater extent.
- Figure 8 shows the device in Figure 5 in a fourth position in which the lower part of the mouthpiece has opened with respect to the position in Figure 5.
- Figure 9 shows an alternative embodiment of the strap connector in accordance with the invention.
- Figure 10 shows an alternative embodiment of the strap in accordance with the invention.
- Figure 11 shows another embodiment of the strap connector in accordance with the invention.
- Figure 12 shows a perspective view of another embodiment of the device in accordance with the invention.
- Figure 13 shows a front elevation view of the device of Figure 12.
- Figures 14 to 17 show four assembly phases of the device of Figure 1.
- Figures 18 to 21 show four assembly phases of the device of Figure 12.
- Figure 22 shows a graph of the relationship between the traction force and the displacement or deformation of an embodiment of the invention having an arched body.

### Detailed Description of the Invention

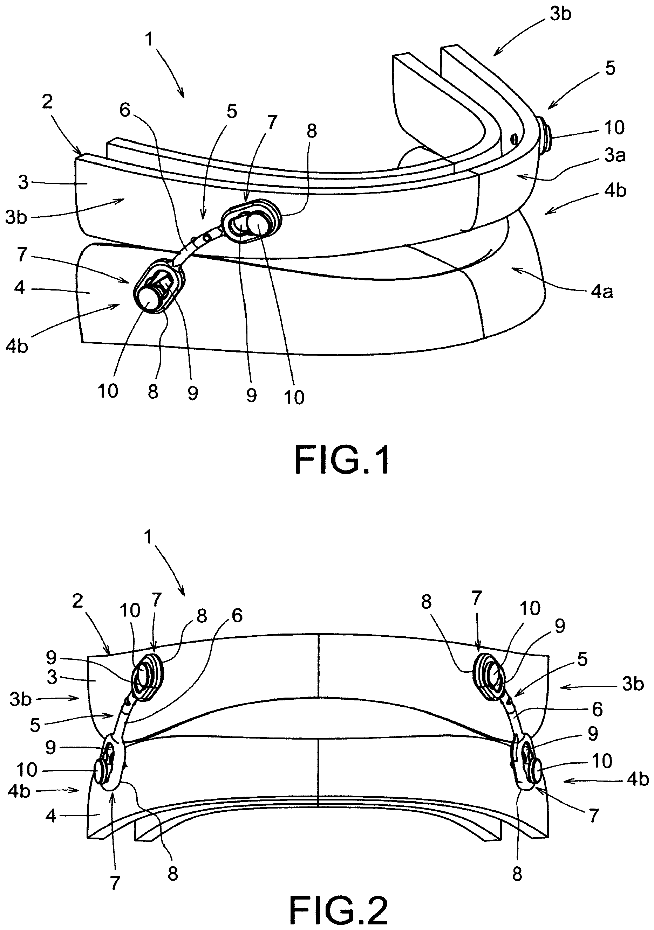

Figures 1 and 2 show a perspective view and a front view of first embodiment of a mandibular advancement device in accordance with the invention. The device (1) of the present embodiment comprises a mouthpiece (2), generally custom-made for the patient, comprising an upper portion (3) intended to be placed on the upper dental arch and a lower portion (4) intended to be placed on the lower dental arch. For example, the mouthpiece (2) may be a bite splint, which is a specific type of mouthpiece designed to treat bruxism, a clinical phenomenon consisting of undesired and excessive grinding and/or clenching of teeth. The mouthpiece may be manufactured with vacuum sheets, using CAD/CAM, by dust apposition, or by any other applicable manufacturing system.

Each portion (3, 4) of the mouthpiece (2) includes a front area (3a, 4a) intended to be placed alongside the user's incisor and canine teeth, and two lateral areas (3b, 4b) intended to be placed in the area of the user's premolar and molar teeth. The device (1) in accordance with the invention and of the present embodiment further includes two plastic straps (5), one on each side of the mouthpiece (2). Each strap (5) comprises an elongate body (6) fitted with a respective connector (8) at each end (7) thereof. Each connector (8) includes a respective slot (9). The body (6) of the present embodiment is a single cord, which is an extremely resistant solution in mechanical terms, is unlikely to break, at can be manufactured easily and at reasonable cost. The slots (9) at the ends (7) of the straps (5) are articulately connected to protrusions (10) on the mouthpiece (2). More specifically, in this embodiment, the slot (9) at the upper end (7) of each strap (5), according to the position in Figure 1, is articulately connected to a protrusion (10) that protrudes from the lateral area (3b) of the upper portion (3), whilst the slot (9) at the lower end (7) is articulately connected to a protrusion (10) that protrudes from the lateral area (4b) of the lower portion (4).

The plastic material used to make the strap (5) should be relatively rigid but should also have certain elasticity to deformation. Examples of suitable materials for the straps are Polyamide 12 or Nylon 6.3, although these examples are not intended to be limiting and use of other materials is not ruled out.

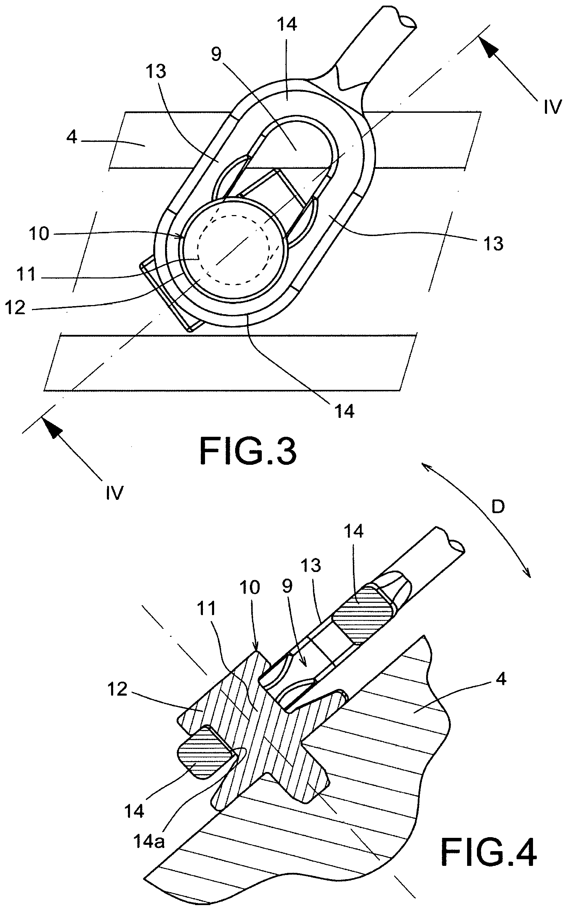

Figure 3 shows a partial left side elevation view of the device (1) of Figure 1, in which only the area of the lower end (7) of the left-hand strap (5) according to the position of the device (1) in Figure 1 is shown. In turn, Figure 4 shows a cross-sectional view of the assembly of Figure 3 as per sectional plan IV-IV. Both figures clearly show that the protrusion (10) comprises a cylindrical portion (11) and a head (12). The head (12) is placed further out and is wider than the cylindrical portion (11), the purpose of this head (12) being to prevent the end (7) of the strap (5) from disconnecting from the protrusion (10). On the other hand, the cylindrical portion (11) is arranged inside the slot (9) of the strap (5). Connecting the slot (9) to a cylindrical-shaped part allows achieving an articulating connection between the strap (5) and the lower portion (4) of the mouthpiece (2), so that the strap (5) acts like an articulated link. The articulated connection is favored by the fact that the connector (8) comprises a closed loop having two straight lateral portions (13) and two curved end portions (14), for example in the shape of an arc of a circle.

In accordance with the invention, the slot (9) is closed and its length is longer than the diameter of the cylindrical portion (11). This enables the cylindrical portion (11) to move longitudinally along the slot (9), as well as to rotate with respect to the slot (9). The usefulness of this longitudinal displacement is illustrated in the following four figures.

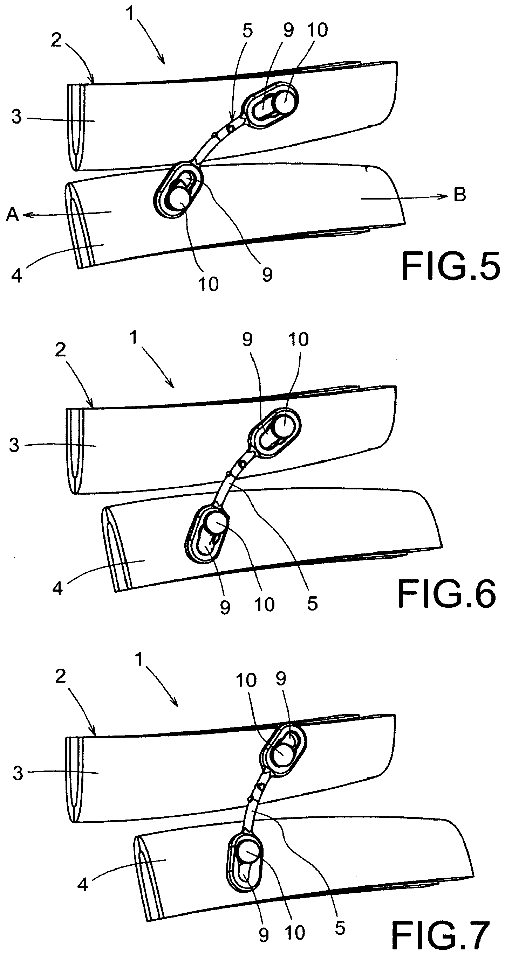

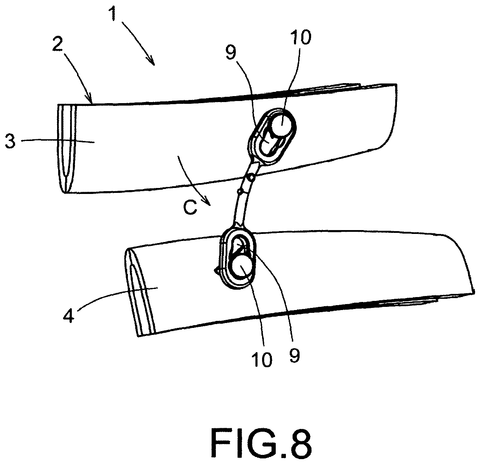

Figures 5 to 8 show various side views of the device (1) of Figure 1 in different positions. Figure 5 shows the device (1) in an initial or rest position, in which the lower portion (4) of the mouthpiece (2) is slightly advanced with respect to the upper portion (3). This position will hereinafter be called the "treatment position", as it is a position that has been designed and planned by a medical professional to provide appropriate mandibular advancement and to open the airways of a certain patient during sleep. In this treatment position, as can be seen in the figure, the protrusions (10) and the strap (5) are positioned in such a way that the strap acts by traction; i.e., if the lower portion (4) tries to move backwards - in the direction indicated by arrow (A)- the strap (5) pulls on the lower portion (4) and prevents such movement. On the other hand, in the treatment position of Figure 5, the device (1) does allow the patient to move the lower portion (4) of the mouthpiece (2) forward, i.e., in the direction indicated by arrow (B), as can be seen in Figures 6 and 7. More specifically, the device allows forward movement because the two slots (9) can move in the direction of arrow (B) with respect to the protrusions (10) that are inside them, from the initial traction position (treatment position of Figure 1). The maximum forward movement distance of the lower portion (4) with respect to the upper portion (3) will essentially depend on the difference between the length of the slots (9) and the diameter of the cylindrical portions (11) of the protrusions (10). When the protrusions (10) have reached the innermost ends of the slots (9) as shown in Figure 7, the strap (5) starts to act by compression, preventing the user from moving the lower portion (4) forward. Furthermore, as can be observed in Figure 8, the device (1) also allows the lower portion (4) to descend with respect to the upper portion (3), indicated by arrow (C). This descending movement is possible due to the fact that the cylindrical portions (11) of the protrusions (10) are articulately connected and can therefore turn inside the slots (9). In short, the device in accordance with the invention ensures the proper treatment of apnea as it prevents the lower portion (4) from moving backwards, whilst it is comfortable for the patient by allowing significant freedom of movement of the lower portion (4) in the forward direction and in the mouth-opening direction.

As can be seen in Figure 4, the end portion (14) of the connector (8) has an interior cylindrical wall (14a) adapted to come into contact with the cylindrical portion (11) of the protrusion (10); this contact makes it difficult for the strap (5) to turn with respect to the protrusion (10) in the direction indicated by the arrow (D) when the strap (5) is acting by traction, i.e., when there is contact between the cylindrical portion (11) and the interior cylindrical wall (14a). In consequence, the patient is able to move his or her mouth laterally only to a certain extent, perceiving a sensation that he or she is leaving the treatment position. Therefore, the device in accordance with the invention also allows sideways mandibular movement, which is comfortable for the patient, but limited. Adjusting the height difference between the cylindrical portion (11) and the interior cylindrical wall (14a) leads to a greater or lesser sensory indication as to the degree of lateral misalignment; anchoring is more rigid when the gap is smaller. The most rigid scenario takes place in the event of a small interference between the length of the cylindrical section and the thickness of the portion.

Furthermore, in accordance with the invention, the body (6) is arched, which provides the strap (5) with certain flexibility when acting by traction. Thus, in the treatment position in Figure 5, if the patient tries to move the lower portion (4) backwards and the strap (5) prevents this movement by acting by traction, the patient will not notice a sudden pull of the strap but rather the pulling effect will be dampened by the elasticity of the arched body (6). In consequence, it will be possible to manufacture devices with different elasticity by varying the curvature of the central cord or the body (6), without undermining the security of the anchor and traction movement guiding. In principle, the greater the curvature, the less rigid the articulated rod or strap (5) is, thus being more comfortable for the patient.

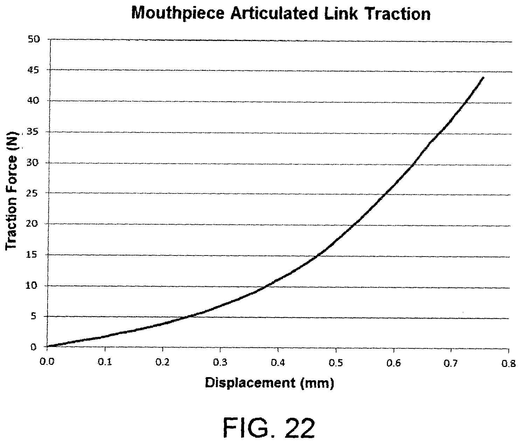

Furthermore, the curvature provides another beneficial effect, whereby when the device acts by traction, the longitudinal elastic force with which the arched body (6) tends to recover its original position increases as the body (6) stretches. In other words, the arched body (6) becomes gradually more rigid. Figure 22 shows a graph of the relationship between the traction force and the movement of one end of the body (6) with respect to the other (in other words, the deformation of the body (6)) for an exemplary device in accordance with the invention. As can be seen, just when the body (6) starts to deform and act by traction, the rigidity is extremely low; as deformation increases, the rigidity increases to a greater extent than the deformation, thus not following a linear relationship. The body (6) is therefore extremely flexible as long as a curvature remains. This curvature starts to disappear as the jaw moves backwards. When the curvature disappears or is notably reduced, the rigidity increases significantly and can be multiplied by 10 or more. This phenomenon causes the appearance of the force that opposes the backward movement of the jaw to take place in an extremely comfortable manner for the patient. When the patient tries to move his/her jaw backwards, he/she does not feel a sharp pull but gradual dampening of this movement.

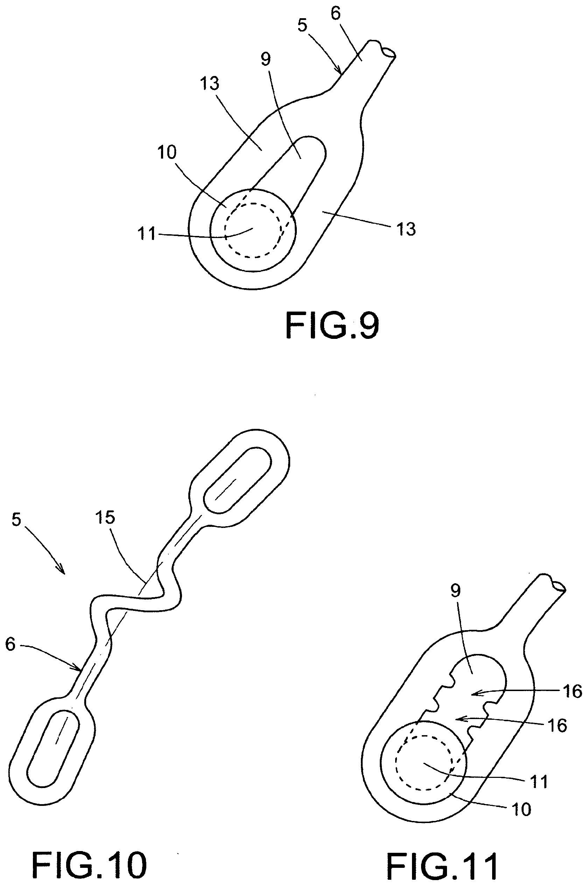

In the method shown in Figure 3, the lateral portions (13) of the connectors (8) are parallel. This permits that the effort required to move the lower portion (4) forward and to move the lower portion (4) back to the treatment position is the same. Alternatively, as can be seen in Figure 9, a different embodiment is possible in which the lateral portions (13) are not parallel but are convergent towards the body (6) of the strap (5). In consequence, the effort required by the patient to move the lower portion (4) forward increases as the cylindrical portion (11) moves forward along an increasingly narrower slot (9). This means that, in this embodiment, while the device does allow the patient to leave the treatment position and move the lower portion (4) forward, it provides an indication to the patient that he or she should return to the treatment position.

Figure 10 shows an additional embodiment of the invention, in which the body (6) is laid along a direction (15) that has at least one inflexion; in particular, in the example shown, the inflexions of the direction provide an S-shape. The purpose of the inflexions is to create points of greater flexibility in the body (6).

Figure 11 shows an additional embodiment of the invention, in which the slot (9) has at least one narrowing (16) - two, in the embodiment shown in the figure - capable of offering resistance to movement of the cylindrical portion (11) of the protrusion (10) throughout the slot (9). Due to the narrowing (16), the patient must exert certain additional force in order for the cylindrical portion (11) to pass through the narrowing (16) and move further along the slot (9). In consequence, the patient perceives a resistance, and even a "click" feeling, as the mandible moves farther from the treatment position, reminding the patient that although there is certain freedom of forward movement for the mandible, it is recommendable to return to the treatment position.

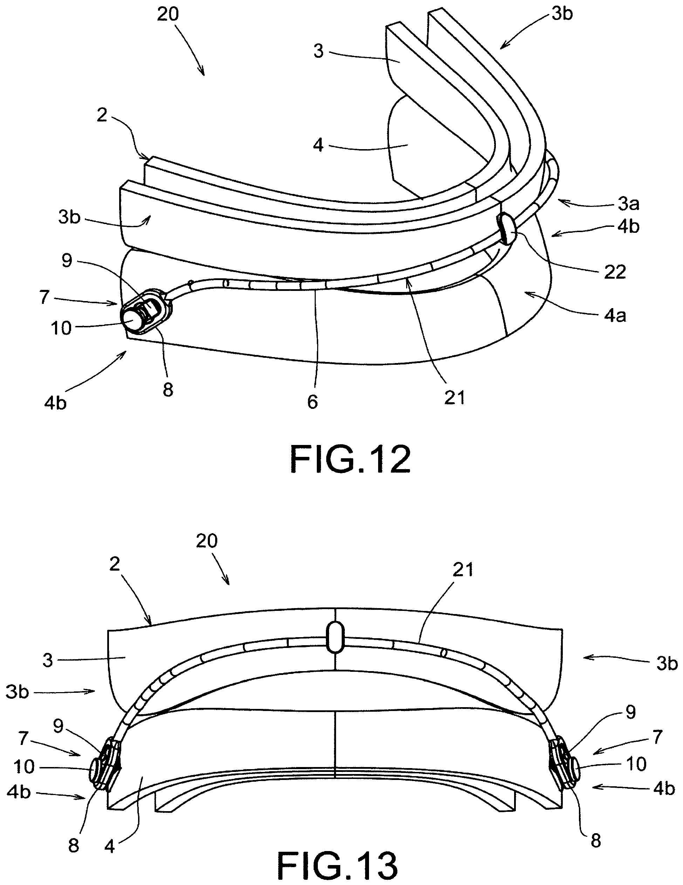

Figures 12 and 13 show a perspective view and a front view of an alternative embodiment of a mandibular advancement device in accordance with the invention. In this case, the device (20) consists of just one strap (21), with slots (9) at the ends (7) as in the embodiment of Figure 1. In the device (20) of the present embodiment, one slot (9) of the strap (21) is articulately connected to a protrusion (10) which protrudes from a lateral area (4b) of the lower portion (4), and the other slot (9) of the strap (21) is articulately connected to a protrusion (10) that protrudes from the opposite lateral area (4b) of the lower portion (4). In addition, the front area (3a) of the upper portion (3) preferably comprises at least one hook (22) that allows free sideways movement of the strap (21) with respect to the upper portion (3) and prevents the strap (21) from separating longitudinally from the upper portion (3). In other words, the connection between the strap (21) and the device (20) in this embodiment is carried out by two articulated connections in the mandible (in the molar or premolar teeth) and a sliding connection in the upper central part. The sliding connection allows that the strap (21) to be laid in a more horizontal position, and therefore the mandibular advancement effort exerted by the strap (21) is more efficient (bearing in mind that vertical forces are irrelevant or parasite in terms of achieving mandibular advancement). Furthermore, the central sliding anchor is extremely comfortable and does not annoy the patient.

An additional advantage of the present embodiment is that it is an isostatic device, i.e., a device that achieves an equal load and force distribution on both sides of the upper maxillary bone and the mandible even when the protrusions (10) are not placed perfectly symmetrically on each side of the lower portion (4) of the mouthpiece (2). This means that this embodiment is independent to accuracy in positioning the protrusions (10). Furthermore, this device allows free lateral movement with a greater amplitude than in the previous embodiment, said lateral movement not having to be necessarily centered (in other words, it does not tend to return to the centered position on its own), which, added to the fact that the efforts are homogenously divided, makes this device extremely comfortable for the patient.

Furthermore, unlike the embodiment in Figure 1, the compression capacity of this embodiment is null, or in other words, it never works by compression (it is only rigid to traction).

A similar embodiment to that of Figure 12 is contemplated, where the hook of the upper portion (3) of the mouthpiece (2) is made in the shape of a seat area for the strap (21). This seat area may be in the form of a recess, step or channel in which the strap (21) is placed and hooked. This offers the user a compact and comfortable single isostatic strap device solution.

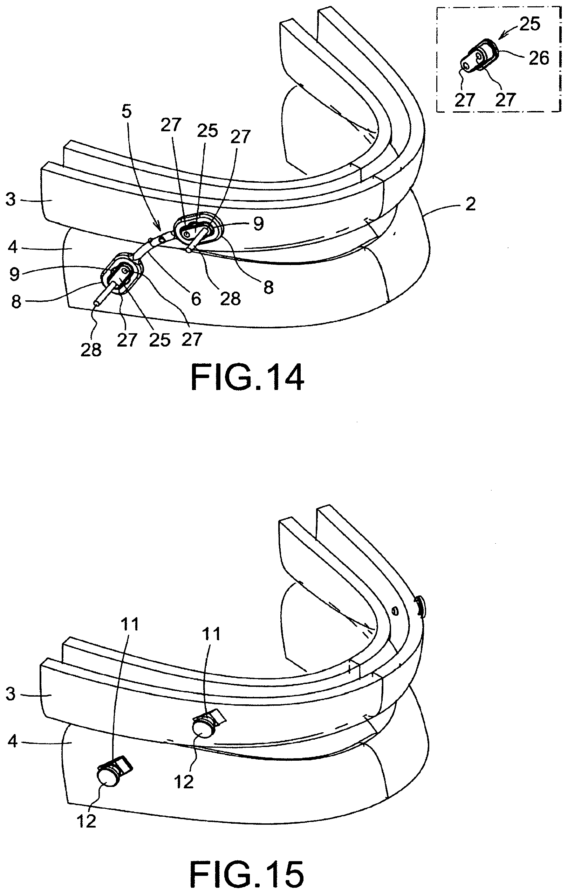

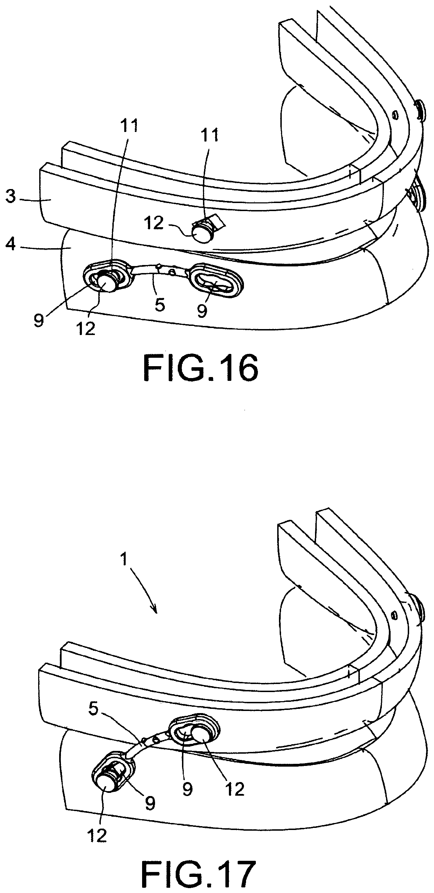

Another object of the disclosure is a preparation method for a mandibular advancement device in accordance with the invention, which allows a mouthpiece (2) to be turned into a device (1; 20) in accordance with the invention. Figures 14 to 17 show an exemplary embodiment of the method for preparing the device (1) of Figure 1. In turn, Figures 17 to 21 show another exemplary embodiment of the method for preparing the device (20) of Figure 12.

With reference to Figures 14 to 17, the preparation method begins with a mouthpiece (2) designed for a specific patient. Initially, as shown in Figure 14, the lower portion (4) of the mouthpiece (2) is positioned underneath the upper portion (3) and slightly forward with respect to the upper portion (3) in accordance with the treatment position previously established by the doctor or other applicable person. Then, the strap (5) is placed in a position as far back as possible (normally so that the slot (9) of the lower connector (8) is positioned in the area of the premolar teeth and the slot (9) of the upper connector (8) is positioned in the area of the canine teeth). Then, a guide part (25) is inserted in each slot (9) of the strap (5). The guide part (25), which has been drawn alone for informative purposes in a separate box on the upper right-hand side of the figure, is a piece with a certain thickness that has an external contour (26) that adapts to the shape of the slot (9) of the strap (5) in accordance with the invention, and that has a through hole (27) in at least one of its two ends. As per the method of the invention, the guide part (25) must be inserted in such a way that one through hole (27) is placed in the outermost end of the slot (9). Since the guide parts (25) in the figure present a through hole (27) at each end, they can be inserted indistinctively in one direction or the other (turned 180°), facilitating the assembly process. Once the guide part (25) has been inserted, a point (28) is inserted in the endmost through hole (27) of each guide part (25), i.e., in the through hole (25) that is farthest away from the body (6) of the strap (5). The points (28) are used to mark the respective drilling points in the upper portion (3) and the lower portion (4) of the mouthpiece (2). Then, the points (28), the guide parts (25) and the strap (5) are removed and the marked points are drilled, forming respective small holes in the upper portion (3) and the lower portion (4). Then, as shown in Figure 15, a pin is placed in each of the small drilled holes and glued with resin. The pins provide a protruding cylindrical portion (11) ending in a wider head (12). Then, as shown in Figures 16 and 17, the strap (5) is positioned by first inserting one pin in one slot (9) and then inserting the other pin in the other slot (9) of the strap (5). The same process is carried out on the other side of the mouthpiece (2) to position a second strap (5). As a result, the mandibular advancement device (1) of Figure 1 is obtained.

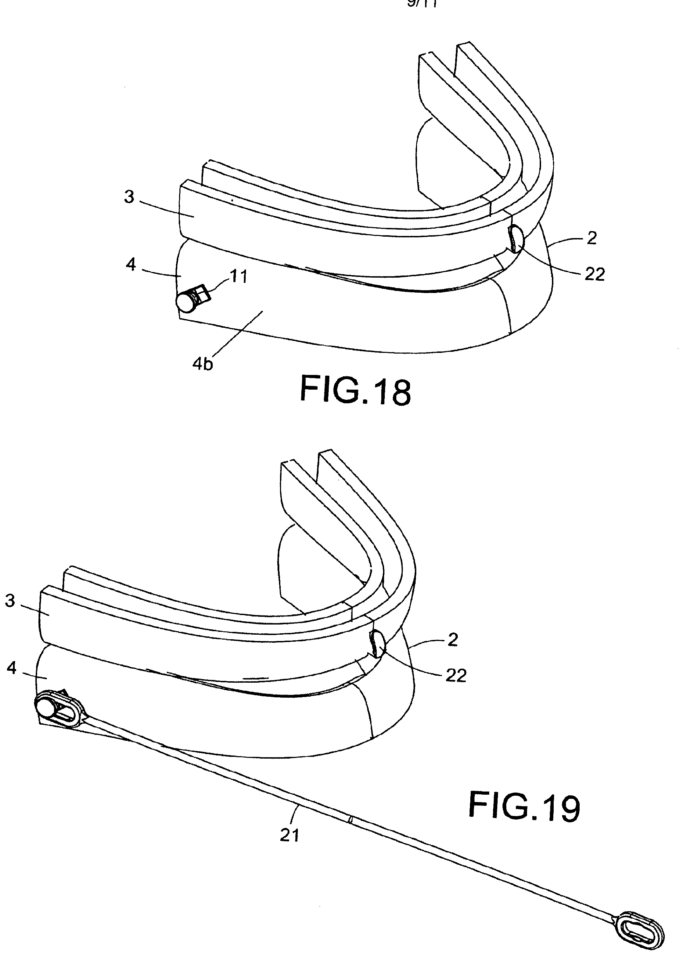

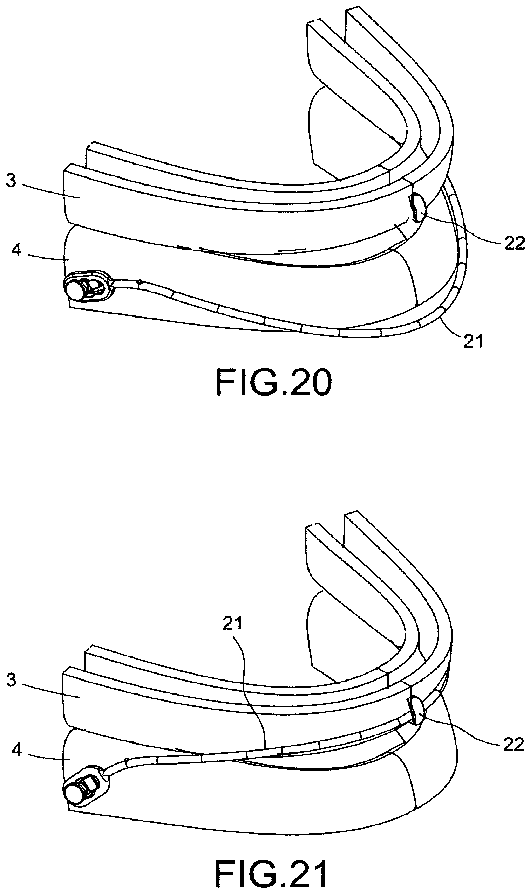

With reference to Figures 18 to 21, the preparation method once again begins with a mouthpiece (2) designed for a certain patient. In this case, however, as shown in Figure 18, a pin is first of all placed in one of the lateral areas (4b) of the lower portion (4), as far back as possible (normally in the molar teeth or even behind the last tooth). This pin provides a cylindrical portion (11) to hook the strap (21). Another pin is also placed in the front area (3a) of the upper portion (3), whereby this second pin provides a hook (22). Then, the strap (21) is hooked in the pin as shown in Figure 19, the strap (21) is extended towards the opposite lateral area (4b) as shown in Figure 20, and the strap (21) is hooked to the upper central pin as shown in Figure 21. Then, using the guide part (25) as already explained, a hole is drilled in the outermost area of the slot (9) which is in the opposite lateral area, hidden in the figure. The strap (21) is removed, a hole is drilled and another pin is glued in this opposite lateral area. In this way, the strap (21) is assembled so that it works by traction, in accordance with the invention, preventing the lower portion (4) of the mouthpiece (2) from moving backwards.

The use of guide parts (25) to position the cylindrical portions (11) of the straps (5; 21) significantly facilitates preparing a device (1; 20) based on a personalized treatment position for each patient, and allows precise positioning of these cylindrical portions (11) and hence of the straps (5; 21). A kit for mandibular advancement treatment may comprise at least one strap (5; 21) and at least one guide part (25). By using this kit and the method disclosed, it is possible to adapt a mouthpiece (2), for example a bite splint, to turn it into a mandibular advancement device (1; 20) in accordance with the invention.

## Claims

1. A mandibular advancement device (1; 20), comprising a mouthpiece (2) made up of an upper portion (3) and a lower portion (4), wherein each portion has a front area (3a, 4a) intended to be placed in the area of the incisor and canine teeth of a user and two lateral areas (3b, 4b) intended to be placed in the area of the user's premolar and molar teeth, comprising:
- at least one plastic strap (5; 21) with two ends (7), each end (7) fitted with a slot (9) articulately connected to a respective cylindrical portion (11) of a protrusion (10) that protrudes laterally from the mouthpiece (2), wherein
- the slot (9) is closed and is of a longer length than the diameter of the cylindrical portion (11), so that the cylindrical portion (11) can move throughout the slot (9), wherein
- the strap (5; 21) is adapted to act by traction between the cylindrical portions (11) of the mouthpiece (2) to prevent a free return of the lower portion (4) with respect to the upper portion (3), and further wherein the strap (5; 21) is in the form of an elongate body (6) fitted with two connectors (8) on both ends (7) in which the respective slots (9) are located,
**characterized in that**
- the body (6) is arched and the strap (5; 21) is flexible and elastically stretchable by an outward pulling on at least one of the two ends (7) thereof, wherein a traction force exerted by the strap (5; 21) on the two ends (7) gradually increases as the curvature of the body (6) disappears and the strap (5; 21) gradually stretches.

2. The device (1) of claim 1, **characterized in that** it comprises wo straps (5), one on each side of the mouthpiece (2), wherein on each strap (5) one slot (9) is articulately connected to a protrusion (10) that protrudes from the lateral area (4b) of the lower portion (4) and the other slot (9) is articulately connected to a protrusion (10) that protrudes from the lateral area (3b) of the upper portion (3).

3. The device (20) of claim 1, **characterized in that** it includes a single strap (21), wherein one slot (9) of said strap (21) is articulately connected to a protrusion (10) that protrudes from the lateral area (4b) of the lower portion (4) and the other slot (9) of said strap (21) is articulately connected to a protrusion (10) that protrudes from the opposite lateral area (4b) of the lower portion (4).

4. The device (20) of claim 3, **characterized in that** the front area (3a) of the upper portion (3) comprises at least one hook (22) which allows free lateral movement of the strap (21) with respect to the upper portion (3) and which prevents the strap (21) from separating longitudinally from the upper portion (3).

5. The device (1; 20) of claim 1, **characterized in that** the connectors (8) comprise a closed loop with two straight lateral portions (13) and two curved end portions (14).

6. The device (1) of claim 5, **characterized in that** the lateral portions (13) are parallel.

7. The device (1) of claim 5, **characterized in that** the lateral portions (13) are convergent towards the body (6).

8. The device (1) of claim 5, **characterized in that** the end portion (14) of the connectors (8) has a cylindrical interior wall (14a) adapted to come into contact with the cylindrical portion (11) of the protrusion (10).

9. The device (1) of claim 5, **characterized in that** the body (6) is a single cord.

10. The device (1) of claim 5, **characterized in that** the body (6) is arranged along a direction (15) with at least one inflection.

11. The device (1) of claim 1, **characterized in that** each slot (9) has at least one narrowing (16) capable of offering resistance to movement of the cylindrical portion (11) of the protrusion (10) along the slot (9).

## Patentansprüche

1. Mandibuläre Vorschubvorrichtung (1; 20), die ein Mundstück (2) umfasst, das aus ein oberes Teil (3) und ein unteres Teil (4) hergestellt ist, wobei jedes Teil einen vorderen Bereich (3a, 4a) aufweist, der vorgesehen ist in dem Bereich der Schneide- und Eckzähne eines Anwenders angeordnet zu werden, und zwei seitliche Bereiche (3b, 4b) aufweist, die vorgesehen sind in dem Bereich der prämolaren und molaren Zähne des Anwenders angeordnet zu werden, umfassend:
- mindestens einen Kunststoffstreifen (5; 21) mit zwei Enden (7), wobei jedes Ende (7) mit einem Schlitz (9) ausgestattet ist, das durch Gelenk mit einem jeweiligen zylindrischen Abschnitt (11) von einem Überstand (10) verbunden ist, der von dem Mundstück (2) seitlich hervorsteht, wobei
- der Schlitz (9) geschlossen ist und von einer größeren Länge als der Durchmesser des zylindrischen Abschnitts (11) ist, so dass der zylindrische Abschnitt (11) sich durchgehend im Schlitz (9) bewegen kann, wobei
- der Streifen (5; 21) angepasst ist, um durch Zug zwischen den zylindrischen Abschnitten (11) des Mundstücks (2) zu wirken, um eine freie Rückkehr des unteren Teils (4) mit Bezug auf das obere Teil (3) zu verhindern, und weiterhin wobei
- der Streifen (5; 21) in Form von einem länglichen Körper (6) vorliegt, ausgestattet mit zwei Verbindern (8) an beiden Enden (7), in denen die jeweiligen Schlitze (9) angeordnet sind, **dadurch gekennzeichnet, dass**
- der Körper (6) gebogen ist und der Streifen (5; 21) durch ein Auswärtsziehen an mindestens einem der zwei Enden (7) davon flexibel und elastisch streckbar ist, wobei eine Zugkraft, ausgeübt durch den Streifen (5; 21) an den zwei Enden (7), allmählich zunimmt, wobei die Krümmung des Körpers (6) verschwindet und der Streifen (5; 21) sich allmählich streckt.

2. Vorrichtung (1) nach Anspruch 1, **dadurch gekennzeichnet, dass** sie zwei Streifen (5) umfasst, einen an jeder Seite des Mundstücks (2), wobei an jedem Streifen (5) ein Schlitz (9) durch Gelenk mit einem Überstand (10) verbunden ist, der von dem seitlichen Bereich (4b) des unteren Teils (4) hervorsteht und der andere Schlitz (9) durch Gelenk mit einem Überstand (10) verbunden ist, der von dem seitlichen Bereich (3b) des oberen Teils (3) hervosteht.

3. Vorrichtung (20) nach Anspruch 1, **dadurch gekennzeichnet, dass** sie einen einzelnen Streifen (21) enthält, wobei ein Schlitz (9) des Streifens (21) durch Gelenk mit einem Überstand (10) verbunden ist, der von dem seitlichen Bereich (4b) des unteren Teils (4) hervorsteht und der andere Schlitz (9) des Streifens (21) durch Gelenk mit einem Überstand (10) verbunden ist, der von dem entgegengesetzten seitlichen Bereich (4b) des unteren Teils (4) hervorsteht.

4. Vorrichtung (20) nach Anspruch 3, **dadurch gekennzeichnet, dass** der vordere Bereich (3a) des oberen Teils (3) mindestens einen Haken (22) umfasst, der die freie seitliche Bewegung des Streifens (21) mit Bezug auf das obere Teil (3) erlaubt und der den Streifen (21) daran hindert, sich längs von dem oberen Teil (3) zu trennen.

5. Vorrichtung (1; 20) nach Anspruch 1, **dadurch gekennzeichnet, dass** die Verbinder (8) eine geschlossene Schlaufe mit zwei geraden seitlichen Abschnitten (13) und zwei gebogenen Endabschnitten (14) umfassen.

6. Vorrichtung (1) nach Anspruch 5, **dadurch gekennzeichnet, dass** die seitlichen Abschnitte (13) parallel sind.

7. Vorrichtung (1) nach Anspruch 5, **dadurch gekennzeichnet, dass** die seitlichen Abschnitte (13) zum Körper hin (6) zusammenlaufen.

8. Vorrichtung (1) nach Anspruch 5, **dadurch gekennzeichnet, dass** der Endabschnitt (14) der Verbinder (8) eine zylindrische Innenwand (14a) aufweist, die angepasst ist um mit dem zylindrischen Abschnitt (11) des Überstands (10) in Kontakt zu kommen.

9. Vorrichtung (1) nach Anspruch 5, **dadurch gekennzeichnet, dass** der Körper (6) eine einzelne Schnur ist.

10. Vorrichtung (1) nach Anspruch 5, **dadurch gekennzeichnet, dass** der Körper (6) entlang einer Richtung (15) mit mindestens einer Biegung angeordnet ist.

11. Vorrichtung (1) nach Anspruch 1, **dadurch gekennzeichnet, dass** jeder Schlitz (9) mindestens eine Verengung (16) aufweist, die in der Lage ist, der Bewegung des zylindrischen Abschnitts (11) des Überstands (10) entlang des Schlitzes (9) Widerstand zu bieten.

## Revendications

1. Dispositif d'avancement mandibulaire (1; 20) comprenant un embout (2) constitué par une portion supérieure (3) et une portion ultérieure (4), où chaque portion a une zone frontale (3a, 4a) destinée à venir se placer dans la zone des incisives et des canines d'un utilisateur et deux zones latérales (3b, 4b) destinées à venir se placer dans la zone des prémolaires et des molaires de l'utilisateur, comprenant:
- au moins une bande (5; 21) plastique présentant deux extrémités (7), chaque extrémité (7) étant munie d'une fente (9) reliée de manière articulée à une portion cylindrique (11) respective d'une protubérance (10) qui fait saillie latéralement par rapport à l'embout (2), où
- la fente (9) est fermée et a une longueur supérieure au diamètre de la portion cylindrique (11) d'une manière telle que la portion cylindrique (11) peut se déplacer à travers toute la fente (9); où
- la bande (5; 21) est conçue pour agir par traction entre les portions cylindriques (11) de l'embout (2) afin d'empêcher un retour libre de la portion inférieure (4) par rapport à la portion supérieure (3); où
- la bande (5; 21) se présente sous la forme d'un corps (6) allongé muni de deux raccords (8) aux deux extrémités (7) dans lesquelles les fentes (9) respectives sont situées; **caractérisé en ce que**:
- le corps (6) est arqué et la bande (5; 21) est flexible et élastiquement extensible via une traction vers l'extérieur sur au moins une de ses deux extrémités (7), où une force de traction exercée par la bande (5; 21) sur les deux extrémités (7) augmente de manière progressive au fur et à mesure que la courbure du corps (6) disparaît et que la bande (5; 21) s'étire de manière progressive.

2. Dispositif (1) selon la revendication 1, **caractérisé en ce qu'**il comprend deux bandes (5), une de chaque côté de l'embout (2), où, sur chaque bande (5), une fente (9) est reliée de manière articulée à une protubérance (10) qui fait saillie par rapport à la zone latérale (4b) de la portion inférieure (4) et l'autre fente (9) est reliée de manière articulée à une protubérance (10) qui fait saillie par rapport à la zone latérale (3b) de la portion supérieure (3).

3. Dispositif (20) selon la revendication 1, **caractérisé en ce qu'**il inclut une bande (21) unique, où une fente (9) de ladite bande (21) est reliée de manière articulée à une protubérance (10) qui fait saillie par rapport à la zone latérale (4b) de la portion inférieure (4) et l'autre fente (9) de ladite bande (21) est reliée de manière articulée à une protubérance (10) qui fait saillie par rapport à la zone latérale opposée (4b) de la portion inférieure (4).

4. Dispositif (20) selon la revendication 3, **caractérisé en ce que** la zone frontale (3a) de la portion supérieure (3) comprend au moins un crochet (22) qui permet un mouvement latéral libre de la bande (21) par rapport à la portion supérieure (3) et qui empêche la bande (21) de se séparer longitudinalement de la portion supérieure (3).

5. Dispositif (1; 20) selon la revendication 1, **caractérisé en ce que** les raccords (8) comprennent une boucle fermée présentant deux portions latérales droites (13) et deux portions terminales courbes (14).

6. Dispositif (1) selon la revendication 5, **caractérisé en ce que** les portions latérales (13) sont parallèles.

7. Dispositif (1) selon la revendication 5, **caractérisé en ce que** les portions latérales (13) convergent en direction du corps (6).

8. Dispositif (1) selon la revendication 5, **caractérisé en ce que** la portion terminale (14) des raccords (8) a une paroi interne cylindrique (14a) conçue pour entrer en contact avec la portion cylindrique (11) de la protubérance (10).

9. Dispositif (1) selon la revendication 5, **caractérisé en ce que** le corps (6) est un cordon unique.

10. Dispositif (1) selon la revendication 5, **caractérisé en ce que** le corps (6) est disposé dans une direction (15) présentant au moins une inflexion.

11. Dispositif (1) selon la revendication 1, **caractérisé en ce que** chaque fente (9) a au moins un rétrécissement (16) capable d'opposer une résistance au mouvement de la portion cylindrique (11) de la protubérance (10) le long de la fente (9).
